Europäisches Patentamt

European Patent Office

Office européen des brevets

(19)

(11) Publication number: **0 389 043**
**A1**

# EUROPEAN PATENT APPLICATION

(12)

(21) Application number: **90200613.9**

(22) Date of filing: **15.03.90**

(51) Int. Cl.5: **C12N 15/31, C12N 15/62, A61K 47/48**

(30) Priority: **22.03.89 US 327214**

(43) Date of publication of application:
**26.09.90 Bulletin 90/39**

(84) Designated Contracting States:
**AT BE CH DE DK ES FR GB GR IT LI LU NL SE**

(71) Applicant: **MERCK & CO. INC.**
**126, East Lincoln Avenue P.O. Box 2000**
**Rahway New Jersey 07065-0900(US)**

(72) Inventor: **Riemen, Mark W.**
**326 Pheasant Run Drive**
**Doylestown, PA 18901(US)**
Inventor: **Stirdivant, Steven M.**
**57 Old New Road**
**Warrington, PA 18976(US)**

(74) Representative: **Hesketh, Alan, Dr. et al**
**European Patent Department Merck & Co.,**
**Inc. Terlings Park Eastwick Road**
**Harlow Essex, CM20 2QR(GB)**

(54) **Production of modified PE40.**

(57) The present invention provides modifications of the $PE_{40}$ domain which eliminate the chemical ambiguities caused by the cysteines in $PE_{40}$. Substitution of other amino acids such as, e.g., alanine for the cysteine residues in $PE_{40}$, or deletion of two or more of the cysteine residues improves the biological and chemical properties of conjugates formed between modified $PE_{40}$ and a targeting agent.

EP 0 389 043 A1

## PRODUCTION OF MODIFIED PE₄₀

### BACKGROUND OF THE INVENTION

Traditional cancer chemotherapy relies on the ability of drugs to kill tumor cells in cancer patients. Unfortunately, these same drugs frequently kill normal cells as well as the tumor cells. The extent to which a cancer drug kills tumor cells rather than normal cells is an indication of the compound's degree of selectivity for tumor cells. One method of increasing the tumor cell selectivity of cancer drugs is to deliver drugs preferentially to the tumor cells while avoiding normal cell populations. Another term for the selective delivery of chemotherapeutic agents to specific cell populations is "targeting". Drug targeting to tumor cells can be accomplished in several ways. One method relies on the presence of specific receptor molecules found on the surface of tumor cells. Other molecules, referred to as "targeting agents", can recognize and bind to these cell surface receptors. These "targeting agents" include, e.g., antibodies, growth factors, or hormones. "Targeting agents" which recognize and bind to specific cell surface receptors are said to target the cells which possess those receptors. For example, many tumor cells possess a protein on their surfaces called the epidermal growth factor receptor. Several growth factors including epidermal growth factor (EGF) and transforming growth factor-alpha (TGF-alpha) recognize and bind to the EGF receptor on tumor cells. EGF and TGF-alpha are therefore "targeting agents" for these tumor cells.

"Targeting agents" by themselves do not kill tumor cells. Other molecules including cellular poisons or toxins can be linked to "targeting agents" to create hybrid molecules that possess both tumor cell targeting and cellular toxin domains. These hybrid molecules function as tumor cell selective poisons by virtue of their abilities to target tumor cells and then kill those cells via their toxin component. Some of the most potent cellular poisons used in constructing these hybrid molecules are bacterial toxins that inhibit protein synthesis in mammalian cells. Pseudomonas exotoxin A (PE-A) is one of these bacterial toxins, and has been used to construct hybrid "targeting - toxin" molecules (U.S. Patent 4,545,985).

PE-A is a 66 kD bacterial protein which is extremely toxic to mammalian cells. The PE-A molecule contains three functional domains: 1.) The amino-terminal binding domain, responsible for binding to a susceptible cell; 2.) The internally located "translocating" domain, responsible for delivery of the toxin to the cytosol; 3.) The carboxy-terminal enzymatic domain, responsible for cellular intoxication. PE-A has been used in the construction of "targeting-toxin" molecules, anti-cancer agents in which the 66 kD molecule is combined with the tumor-specific "targeting agent" (monoclonal antibody or growth factor). The "targeting-toxin" molecules produced in this manner have enhanced toxicity for cells possessing receptors for the "targeting agent".

A problem with this approach is that the PE-A antibody or growth factor hybrid still has a reasonably high toxicity for normal cells. This toxicity is largely due to the binding of the hybrid protein to cells through the binding domain of the PE-A. In order to overcome this problem, a protein was recombinantly produced which contains only the enzymatic and "translocating" domains of Pseudomonas exotoxin A (Hwang et. al., Cell, 48:129-137 1987). This protein was named PE₄₀ since it has a molecular weight of 40 kD. PE₄₀ lacks the binding domain of PE-A, and is unable to bind to mammalian cells. Thus, PE₄₀ is considerably less toxic than the intact 66 kD protein. As a result, hybrid "targeting-toxin" molecules produced with PE₄₀ were much more specific in their cellular toxicity (Chaudhary et al., Proc. Nat. Acad. Sci. USA, 84: 4583-4542 1987).

While working with PE₄₀, it was found that the cysteine residues at positions 265, 287, 372 and 379 (numbering from the native 66 kD PE-A molecules; Gray et al., Proc. Natl. Acad. Sci., USA, 81, 2645-2649 (1984)) interfered with the construction of "targeting-toxin" molecules using chemical conjugation methods. The reactive nature of the disulfide bonds that these residues form leads to ambiguity with regard to the chemical integrity of the product "targeting toxin".

### DISCLOSURE STATEMENT

1. U.S. patent 4,545,985 teaches that pseudomonas exotoxin A can be conjugated to antibodies or to epidermal growth factor. Patent 4,545,985 further teaches that these conjugates can be used to kill human tumor cells.

2. U.S. patent 4,664,911 teaches that antibodies can be conjugated to the A chain or the B chain of ricin which is a toxin obtained from plants. Patent 4,664,911 further teaches that these conjugates can be used to kill human tumor cells.

3. U.S. patent 4,675,382 teaches that hormones such as melanocyte stimulating hormone (MSH) can be linked to a portion of the diphtheria toxin protein via peptide bonds. Patent 4,675,382 further teaches that the genes which encode these proteins can be joined together to direct the synthesis of a hybrid fusion protein using recombinant DNA techniques. This fusion protein has the ability to bind to cells that possess MSH receptors.

4. Murphy et al., PNAS USA 83:8258-8262 1986, Genetic construction, expression, and melanoma-selective cytotoxicity of a diphtheria toxin-related alpha-melanocyte-stimulating hormone fusion protein. This article teaches that a hybrid fusion protein produced in bacteria using recombinant DNA technology and consisting of a portion of the diphtheria toxin protein joined to alpha-melanocyte-stimulating hormone will bind to and kill human melanoma cells.

5. Kelley et al., PNAS USA 85:3980-3984 1988, Interleukin 2-diphtheria toxin fusion protein can abolish cell-mediated immunity in vivo. This article teaches that a hybrid fusion protein produced in bacteria using recombinant DNA technology and consisting of a portion of the diphtheria toxin protein joined to interleukin 2 functions in nude mice to suppress cell mediated immunity.

6. Allured et al., PNAS USA 83:1320-1324 1986, Structure of exotoxin A of Pseudomonas aeruginosa at 3.0 Angstrom. This article teaches the three dimensional structure of the pseudomonas exotoxin A protein.

7. Hwang et al., Cell 48:129-136 1987, Functional Domains of Pseudomonas Exotoxin Identified by Deletion Analysis of the Gene Expressed in E. Coli. This article teaches that the pseudomonas exotoxin A protein can be divided into three distinct functional domains responsible for: binding to mammalian cells, translocating the toxin protein across lysosomal membranes, and ADP ribosylating elongation factor 2 inside mammalian cells. This article further teaches that these functional domains correspond to distinct regions of the pseudomonas exotoxin A protein.

8. European patent application 0 261 671 published 30 March 1988 teaches that a portion of the pseudomonas exotoxin A protein can be produced which lacks the cellular binding function of the whole pseudomonas exotoxin A protein but possesses the translocating and ADP ribosylating functions of the whole pseudomonas exotoxin A protein. The portion of the pseudomonas exotoxin A protein that retains the translocating and ADP ribosylating functions of the whole pseudomonas exotoxin A protein is called pseudomonas exotoxin - 40 or PE-40. PE-40 consists of amino acid residues 252-613 of the whole pseudomonas exotoxin A protein as defined in Gray et al., PNAS USA 81:2645-2649 1984. This patent application further teaches that PE-40 can be linked to transforming growth factor-alpha to form a hybrid fusion protein produced in bacteria using recombinant DNA techniques.

9. Chaudhary et al., PNAS USA 84:4538-4542 1987, Activity of a recombinant fusion protein between transforming growth factor type alpha and Pseudomonas exotoxin. This article teaches that hybrid fusion proteins formed between PE-40 and transforming growth factor-alpha and produced in bacteria using recombinant DNA techniques will bind to and kill human tumor cells possessing epidermal growth factor receptors.

10. Bailon et al., Biotechnology, pp. 1326-1329 Nov. 1988. Purification and Partial Characterization of an Interleukin 2-Pseudomonas Exotoxin Fusion Protein. This article teaches that hybrid fusion proteins formed between PE-40 and interleukin 2 and produced in bacteria using recombinant DNA techniques will bind to and kill human cell lines possessing interleukin 2 receptors.

## OBJECTS OF THE INVENTION

It is an object of the present invention to provide modifications of $PE_{40}$ which provide improved chemical integrity and defined structure of conjugate molecules formed between "targeting agents" and modified $PE_{40}$. It is another object of this invention to provide a method for preparing and recovering the modified $PE_{40}$ domain from fusion proteins formed between "targeting agents" and modified $PE_{40}$. These and other objects of the present invention will be apparent from the following description.

## SUMMARY OF THE INVENTION

The present invention provides modifications of the $PE_{40}$ domain which eliminate the chemical ambiguities caused by the cysteines in $PE_{40}$. Substitution of other amino acids such as, e.g., alanine for the cysteine residues in $PE_{40}$, or deletion of two or more of the cysteine residues improves the biological and chemical properties of the conjugates formed between modified $PE_{40}$ and a targeting agent.

3

DETAILED DESCRIPTION OF TEE INVENTION

Hybrid molecules produced by conjugation of EGF and $PE_{40}$ are characterized in three primary assay systems. These assays include: 1 - ADP ribosylation of elongation factor 2 which measures the enzymatic activity of EGF-$PE_{40}$ which inhibits mammalian protein synthesis, 2 - inhibition of radiolabled EGF binding to the EGF receptor on membrane vesicles from A431 cells which measures the EGF receptor binding activity of EGF-$PE_{40}$, and 3 -cell viability as assessed by conversion of 3-[4,5-dimethylthiazol-2-yl]-2,5-diphenyltetrazolium bromide (MTT) to formazan which is used to measure the survival of tumor cells following exposure to EGF-$PE_{40}$. These assays are performed as previously described (Chung et al., Infection and Immunity, 16:832-841 1977, Cohen et al., J. Biol. Chem., 257:1523-1531 1982, Riemen et al., Peptides 8:877-885 1987, Mossman, J. Immunol. Methods, 65:55-63 1983).

To create EGF-$PE_{40}$ protein conjugates with defined chemical structure and superior biologic characteristics, we used recombinantly expressed TGF-alpha - $PE_{40}$ molecules as the source material for modified $PE_{40}$. We first produced a series of recombinant DNA molecules that encoded either TGF-alpha - $PE_{40}$ or specifically modified versions of TGF-alpha - $PE_{40}$. The original or parental TGF-alpha - $PE_{40}$ gene was molecularly cloned in a bacterial TAC expression plasmid vector (pTAC TGF57-PE40) using distinct segments of cloned DNA as described in Example 1. The pTAC TGF57-PE40 DNA clone was used as the starting reagent for constructing specifically modified versions of TGF-alpha - $PE_{40}$ DNA. The specific modifications of the pTAC TGF57-PE40 DNA involve site specific mutations in the DNA coding sequence required to replace two or four of the cysteine codons within the $PE_{40}$ domain of the pTAC TGF57-PE40 DNA with codons for other amino acids. Alternatively, the site specific mutations can be engineered to delete two or four of the cysteine codons within the PE40 domain of pTAC TGF57-PE40. The site specific mutations in the pTAC TGF57-PE40 DNA were constructed using the methods of Winter et al., Nature 299:756-758 1982. Specific examples of the mutated pTAC TGF57-PE40 DNAs are presented in Example 2.

The amino acid sequence of the parent TGF-alpha - $PE_{40}$ is presented in Table 3. The four cysteine residues in the $PE_{40}$ domain of the parental TGF-alpha - $PE_{40}$ hybrid fusion protein are designated residues $Cys^{265}$, $Cys^{287}$, $Cys^{372}$, and $Cys^{379}$. Amino acid residues are numbered as defined for the native 66 kD PE-A molecule (Gray et at., Proc. Natl. Acad. Sci., USA, 81, 2645-2649 1984). The modified TGF-alpha - $PE_{40}$ fusion proteins used to generate the modified $PE_{40}$ molecules contain substitutions or deletions of residues [$Cys^{265}$ and $Cys^{287}$] or [$Cys^{372}$ and $Cys^{379}$], or [$Cys^{265}$, $Cys^{287}$, $Cys^{372}$, and $Cys^{379}$]. To simplify the nomenclature for the modified $PE_{40}$ molecules generated from the modified fusion proteins, we have designated the amino acid residues at positions 265 and 287 as the "A" locus, and the residues at positions 372 and 379 the "B" locus. When cysteines are present at amino acid residues 265 and 287 as in the parental TGF-alpha -$PE_{40}$ fusion protein, the locus is capitalized (i.e. "A:). When the cysteines are substituted with other amino acids or deleted from residues 265 and 287, the locus is represented by a lower case "a". Similarly, when the amino acid residues at positions 372 and 379 are cysteines, the locus is represented by an upper case "B" while a lower case "b" represents this locus when the amino acid residues at positions 372 or 379 are substituted with other amino acids or deleted. Thus when all four cysteine residues in the $PE_{40}$ domain are substituted with alanines or deleted the modified $PE_{40}$ is designated $PE_{40}$ ab. In a similar fashion the parental $PE_{40}$ derived from the parental TGF-alpha - $PE_{40}$ fusion protein with cysteines at amino acid residue positions 265, 287, 372, and 379 can be designated $PE_{40}$ AB.

The source materials (i.e. the TGF-alpha -$PE_{40}$ AB hybrid protein, and the modified TGF-alpha - $PE_{40}$ Ab, aB and ab hybrid proteins), are produced in E. coli using the TAC expression vector system described by Linemeyer et al., Biotechnology 5:960-965 1987. The source proteins produced in these bacteria are harvested and purified by lysing the bacteria in guanidine hydrochloride followed by the addition of sodium sulfite and sodium tetrathionate. This reaction mixture is subsequently dialzyed and urea is added to solubilize proteins which have precipitated from solution. The mixture is centrifuged to remove insoluble material and the recombinant hybrid TGF-alpha - $PE_{40}$ source proteins are separated using ion exchange chromatography, followed by size exclusion chromatography, followed once again by ion exchange chromatography.

Since the single methionine residue in the hybrid source proteins is located between the TGF-alpha and $PE_{40}$ domains, treatment with CNBr would cleave the source proteins, yielding the modified $PE_{40}$ proteins and TGF-alpha. The purified S-sulfonate derivatives of TGF-alpha - $PE_{40}$ are thus subjected to CNBr treatment to remove the TGF portion of the molecule. The desired modified $PE_{40}$ portion is purified by ion-exchange chromatography followed by size exclusion chromatography. The purified modified $PE_{40}$ is then derivatized with a suitable heterobifunctional reagent, e.g. SPDP, to allow conjugation of the desired targeting agent. Following conjugation, size exclusion chromatography is used to isolate the conjugate from non-conjugated materials. Once the purified conjugate is isolated, it is tested for biologic activity using the ADP-ribosylation assay and the relevant receptor binding and cell viability assays.

The following examples illustrate the present invention without, however, limiting the same thereto. All of the enzymatic reactions required for molecular biology manipulations, unless otherwise specified, are carried out as described in Maniatis et al., (1982) In: Molecular Cloning: A Laboratory Manual, Cold Spring Harbor Press.

Example 1

Construction of recombinant DNA clones containing TGF-alpha - $PE_{40}$ DNA

The TGF-alpha DNA segment was constructed using three sets of synthetic oligonucleotides as described by Defeo-Jones et al., Molecular and Cellular Biology 8:2999-3007 1988. This synthetic TGF-alpha gene was cloned into pUC-19. DNA from the pUC-19 clone containing recombinant human TGF-alpha was digested with Sph I and Eco RI. The digestion generated a 2.8 kb DNA fragment containing all of pUC-19 and the 5' portion of TGF-alpha. The 2.8 kb fragment was purified and isolated by gel electrophoresis. An Eco RI to Sph I oligonucleotide cassette was synthesized. This synthetic cassette had the sequence indicated below:

$$5'-CGGACCTCCTGGCTGCGCATCTAGG-3'$$
$$3'-GTACGCCTGGAGGACCGACGCGTAGATCCTTAA-5'$$

For convenience, this oligonucleotide cassette was named 57. Cassette 57 was annealed and ligated to the TGF-alpha containing 2.8 kb fragment forming a circularized plasmid. Clones which contained the cassette were identified by hybridization to radiolabeled cassette 57 DNA. The presence of human TGF-alpha was confirmed by DNA sequencing. Sequencing also confirmed the presence of a newly introduced Fsp I site at the 3' end of the TGF-alpha sequence. This plasmid, named TGF-alpha-57/pUC-19, was digested with HinD III and Fsp I which generated a 168 bp fragment containing the TGF-alpha gene (TGF-alpha-57). A separate preparation of pUC-19 was digested with HinD III and Eco RI which generated a 2.68 kb pUC-19 vector DNA. The $PE_{40}$ DNA was isolated from plasmid pVC 8 (Chaudhary et al., PNAS USA 84:4538-4542 1987). pVC 8 was digested using Nde I. A flush end was then generated on this DNA by using the standard conditions of the Klenow reaction (Maniatis et al., supra, p.113). The flush-ended DNA was then subjected to a second digestion with Eco RI to generate a 1.3 kb Eco RI to Nde I (flush ended) fragment containing $PE_{40}$. The TGF-alpha-57 HinD III to Fsp I fragment (168 bp) was ligated to the 2.68 kb pUC-19 vector. Following overnight incubation, the 1.3 kb EcoRI to Nde I (flush ended) $PE_{40}$ DNA fragment was added to the ligation mixture. This second ligation was allowed to proceed overnight. The ligation reaction product was then used to transform JM 109 cells. Clones containing TGF-alpha-57 $PE_{40}$ in pUC-19 were identified by hybridization to radiolabeled TGF-alpha-57 $PE_{40}$ DNA and the DNA from this clone was isolated. The TGF-alpha-57 $PE_{40}$ was removed from the pUC-19 vector and transferred to a TAC vector system described by Linemeyer et al., Bio-Technology 5:960-965 1987). The TGF-alpha-57 $PE_{40}$ in pUC-19 was digested with HinD III and Eco RI to generate a 1.5 kb fragment containing TGF-alpha-57 $PE_{40}$. A flush end was generated on this DNA fragment using standard Klenow reaction conditions (Maniatis et al., op. cit.). The TAC vector was digested with HinD III and Eco RI. A flush end was generated on the digested TAC vector DNA using standard Klenow reaction conditions (Maniatis et al., op. cit. The 2.7 kb flush ended vector was isolated using gel electrophoresis. The flush ended TGF-alpha-57 $PE_{40}$ fragment was then ligated to the flush ended TAC vector. The plasmid generated by this ligation was used to transform JM 109 cells. Candidate clones containing TGF-alpha-57 $PE_{40}$ were identified by hybridization as indicated above and sequenced. The clone containing the desired construction was named pTAC TGF57-PE40. The plasmid generated by these manipulations is depicted in Table 1. The nucleotide sequence of the amino acid codons of the TGF-alpha -$PE_{40}$ fusion protein encoded in the pTAC TGF-57-PE40 DNA are depicted in Table 2. The amino acid sequence encoded by the TGF-57-PE40 gene is shown in Table 3.

## Example 2

Construction of modified versions of recombinant TGF-alpha - PE₄₀ containing DNA clones: Substitution of alanines for cysteines.

TGF-alpha - PE₄₀ aB:

The clone pTAC TGF57-PE40 was digested with SphI and BamHI and the 750 bp SphI-BamHI fragment (specifying the C-terminal 5 amino acids of TGF-alpha and the N-terminal 243 amino acids of PE₄₀) was isolated. M13 mp19 vector DNA was cut with SphI and BamHI and the vector DNA was isolated. The 750 bp SphI-BamHI TGF-alpha - PE₄₀ fragment was ligated into the M13 vector DNA overnight at 15°C. Bacterial host cells were transformed with this ligation mixture, candidate clones were isolated and their plasmid DNA was sequenced to insure that these clones contained the proper recombinant DNAs. Single stranded DNA was prepared for mutagenesis.

An oligonucleotide (oligo #132) was synthesized and used in site directed mutagenesis to introduce a HpaI site into the TGF-alpha - PE₄₀ DNA at amino acid position 272 of PE₄₀:

5' CTGGAGACGTTAACCCGTC 3' (oligo #132)

One consequence of this site directed mutagenesis was the conversion of residue number 272 in PE₄₀ from phenylalanine to leucine. The mutagenesis was performed as described by Winter et al., Nature, 299:756-758 1982.

A candidate clone containing the newly created HpaI site was isolated and sequenced to validate the presence of the mutated genetic sequence. This clone was then cut with SphI and SalI. A 210 bp fragment specifying the C-terminal 5 amino acids of TGF-alpha and the N-terminal 70 amino acids of PE₄₀ and containing the newly introduced HpaI site was isolated and subcloned back into the parent pTAC TGF57-PE40 plasmid at the SphI-SalI sites. Bacterial host cells were transformed, a candidate clone was isolated and its plasmid DNA was sequenced to insure that this clone contained the proper recombinant DNA. For convenience this clone was named pTAC TGF57-PE40-132. pTAC TGF57-PE40-132 was digested with SphI and HpaI and a 3.96 Kb DNA fragment was isolated. A synthetic oligonucleotide cassette (oligo #153) spanning the C-terminal 5 amino acids of TGF-alpha and the N-terminal 32 amino acids of PE₄₀ and containing SphI and HpaI compatible ends was synthesized and ligated to the digested pTAC TGF57-PE40-132:

```
5'      CGGACCTCCTGGCCATGGCCGAAGAGGGCGGCAGCCTGGCCGCGCTGACCGCGCA
3' GTACGCCTGGAGGACCGGTACCGGCTTCTCCCGCCGTCGGACCGGCGCGACTGGCGCGT


CCAGCTGCACACCTGCCGCTGGAGACGTT 3'
GGTCGACGTGTGGACGGCGACCTCTGCAA 5'      (oligo #153)
```

This oligonucleotide cassette incorporated a change in the TGF-alpha - PE₄₀ DNA so that the codon specifying cysteine at residue 265 now specified alanine. For convenience this plasmid DNA was called pTAC TGF57-PE40-132,153. Bacterial host cells were transformed with pTAC TGF57-PE40-132,153 DNA. Candidate clones were identified by hybridization, isolated and their plasmid DNA was sequenced to insure that it contained the proper recombinant DNA.

pTAC TGF57-PE40-132,153 DNA was digested with HpaI and SalI and a 3.95 Kb vector DNA was isolated. A synthetic oligonucleotide cassette (oligo #142) spanning amino acid residues 272 to 309 of PE₄₀ and containing HpaI and SalI compatible ends was synthesized and ligated to the 3.95 Kb pTAC TGF/PE40 132,153 DNA.

```
5' AACCCGTCATCGCCAGCCGCGCGGCTGGGAACAACTGGAGCAGGCTGGCTATCCGGTGC

3' TTGGGCAGTAGCGGTCGGCGCGCCGACCCTTGTTGACCTCGTCCGACCGATAGGCCACG


AGCGGCTGGTCGCCCTCTACCTGGCGGCGCGGCTGTCGTGGAACCAGG 3'

TCGCCGACCAGCGGGAGATGGACCGCCGCGCCGACAGCACCTTGGTCCAGCT 5' (oligo #142)
```

This oligonucleotide cassette changes the codon specifying cysteine at residue 287 so that this codon now specifies alanine. For convenience this mutated plasmid DNA was called pTAC TGF57-PE40-132,153,142. Bacterial host cells were transformed with this plasmid and candidate clones were identified by hybridization. These clones were isolated and their plasmid DNA was sequenced to insure that it contained the proper recombinant DNA. The pTAC TGF57-PE40-132,153,142 plasmid encodes the TGF-alpha - $PE_{40}$ variant with both cysteines at locus "A" replaced by alanines. Therefore, following the nomenclature described previously this modified version of TGF-alpha - $PE_{40}$ is called TGF-alpha -$PE_{40}$ aB. The amino acid sequence encoded by the TGF-alpha-$PE_{40}$ aB gene is shown in Table 4.


TGF-alpha - $PE_{40}$ Ab:

The clone pTAC TGF57-PE40 was digested with SphI and BamHI and the 750 bp SphI-BamHI fragment (specifying the C-terminal 5 amino acids of TGF-alpha and the N-terminal 252 amino acids of $PE_{40}$) was isolated. M13 mp19 vector DNA was cut with SphI and BamHI and the vector DNA was isolated. The 750 bp SphI-BamHI TGF-alpha - $PE_{40}$ fragment was ligated into the M13 vector DNA overnight at 15°C. Bacterial host cells were transformed with this ligation mixture, candidate clones were isolated and their plasmid DNA was sequenced to insure that these clones contained the proper recombinant DNAs. Single stranded DNA was prepared for mutagenesis.

An oligonucleotide (oligo #133) was synthesized and used in site directed mutagenesis to introduce a BstEII site into the TGF-alpha - $PE_{40}$ DNA at amino acid position 369 of $PE_{40}$:

5' GACGTGGTGACCCTGAC 3' (oligo #133)

One consequence of this mutagenesis was the conversion of the serine residue at position 369 of $PE_{40}$ to a threonine.

A DNA clone containing the newly created BstEII site was identified, isolated and sequenced to ensure the presence of the proper recombinant DNA. This clone was next digested with ApaI and SalI restriction enzymes. A 120 bp insert DNA fragment containing the newly created BstEII site was isolated and ligated into pTAC TGF57-PE40 that had also been digested with ApaI and SalI. Bacterial host cells were transformed, and a candidate clone was isolated and sequenced to insure that the proper recombinant DNA was present. This newly created plasmid DNA was called pTAC TGF57-PE40-133. It was digested with BstEII and ApaI and 2.65 Kb vector DNA fragment was isolated.

A BstEII to ApaI oligonucleotide cassette (oligo #155) was synthesized which spanned the region of TGF-alpha - $PE_{40}$ deleted from the pTAC TGF57-PE40-133 clone digested with BstEII and ApaI restriction enzymes. This cassette also specified the nucleotide sequence for BstEII and ApaI compatible ends.


```
5' GTGACCCTGACCGCGCCGGTCGCCGCCGGTGAAGCTGCGGGCC 3'

    3' GGACTGGCGCGGCCAGCGGCGGCCACTTCGACGC 5'   (oligo #155)
```

This oligonucleotide cassette changed the codons for cysteines at residues 372 and 379 of $PE_{40}$ to codons specifying alanines. Oligonucleotide cassette #155 was ligated to the 2.65 Kb vector DNA fragment. Bacterial host cells were transformed and candidate clones were isolated and sequenced to insure that the proper recombinant DNA was present. This newly created DNA clone was called pTAC TGF57-PE40-133,155. It encodes the TGF-alpha -$PE_{40}$ variant with both cysteines at locus "B" replaced by alanines. Therefore, following the nomenclature described previously this modified version of TGF-alpha - $PE_{40}$ is

called TGF-alpha -PE$_{40}$ Ab. The amino acid sequence encoded by the TGF-alpha-PE$_{40}$ Ab gene is shown in Table 5.

TGF-alpha - PE$_{40}$ ab:

The pTAC-TGF57-PE40-132,153,142 plasmid encoding TGF-alpha - PE$_{40}$ aB was digested with SalI and ApaI and the resultant 3.8 Kb vector DNA fragment was isolated. The pTAC TGF57-PE40-133,155 plasmid encoding TGF-alpha - PE$_{40}$ Ab was also digested with SalI and ApaI and the resultant 140 bp DNA fragment containing the cysteine to alanine changes at amino acid residues 372 and 379 of PE$_{40}$ was isolated. These two DNAs were ligated together and used to transform bacterial host cells. Candidate clones were identified by hybridization with a radiolabeled 140 bp DNA from pTAC TGF57-PE40-133,155. Plasmid DNA from the candidate clones was isolated and sequenced to insure the presence of the proper recombinant DNA. This newly created DNA clone was called pTAC TGF57-PE40-132,153,142,133,155. This plasmid encodes the TGF-alpha - PE$_{40}$ variant with all four cysteines at loci "A" and "B" replaced by alanines. Therefore, following the nomenclature described previously this modified version of TGF-alpha -PE$_{40}$ is called TGF-alpha - PE$_{40}$ ab. The amino acid sequence encoded by the TGF-alpha-PE$_{40}$ ab gene is shown in Table 6.

## Example 3

## Production and isolation of recombinant TGF-alpha -PE$_{40}$ source proteins

Transformed E. coli JM-109 cells were cultured in 1 L shake flasks in 500 mL LB-Broth in the presence of 100 ug/mL ampicillin at 37° C. After the A$_{600}$ spectrophotometric absorbance value reached 0.6, isopropyl B-D-thiogalactopyranoside was added to a final concentration of 1 mM. After 2 hours the cells were harvested by centrifugation.

The cells were lysed in 8 M guanidine hydrochloride, 50 mM Tris, 1 mM EDTA, pH 8.0 by stirring at room temperature for 2 hours. The lysis mixture was brought to 0.4 M sodium sulfite and 0.1 M sodium tetrathionate by adding solid reagents and the pH was adjusted to 9.0 with 1 M NaOH. The reaction was allowed to proceed at room temperature for 16 hours.

The protein solution was dialysed against a 10,000 fold excess volume of 1 mM EDTA at 4° C. The mixture was then brought to 6 M urea, 50 mM NaCl, 50 mM Tris, pH 8.0, at room temperature and stirred for 2 hours. Any undissolved material was removed by centrifugation at 32,000 x g for 30 minutes.

The cleared supernatant from the previous step was applied to a 26 x 40 cm DEAE Sepharose Fast-Flow column (Pharmacia LKB Biotechnology, Inc.) equilibrated with 6 M urea, 50 mM Tris, 50 mM NaCl, pH 8.0, at a flow rate of 1 mL/minute. The column was washed with the equilibration buffer until all unadsorbed materials were removed as evidenced by a UV A$_{280}$ spectrophotometric absorbance below 0.1 in the equilibration buffer as it exits the column. The adsorbed fusion protein was eluted from the column with a 1000 mL 50-350 mM NaCl gradient and then concentrated in a stirred cell Amicon concentrator fitted with a YM-30 membrane.

The concentrated fusion protein (8 mL) was applied to 2.6 x 100 cm Sephacryl S-300 column (Pharmacia LKB Biotechnology, Inc.) equilibrated with 6 M urea, 50 mM Tris, 50 mM NaCl, pH 8.0, at a flow rate of 0.25 mL/minute. The column was eluted with additional equilibration buffer and 3 mL fractions collected. Fractions containing TGF-alpha - PE$_{40}$ activity were pooled.

The pooled fractions from the S-300 column were applied to a 1.6 x 40 cm Q Sepharose Fast-Flow column (Pharmacia LKB Biotechnology, Inc.) equilibrated with 6 M urea, 50 mM Tris, 50 mM NaCl, pH 8.0 at a flow rate of 0.7 mL/minute. The column was washed with the equilibration buffer and then eluted with a 600 mL 50-450 mM NaCl gradient. The fractions containing the TGF-alpha - PE$_{40}$ activity were pooled and then dialyzed against 50 mM glycine pH 9.0 and stored at -20° C.

## Example 4

CNBR cleavage of TGF-alpha - PE$_{40}$ source proteins and isolation of modified PE$_{40}$s (PE$_{40}$ AB, PE$_{40}$ Ab, PE$_{40}$ aB, PE$_{40}$ab).

The desired fusion protein, still in the S-sulfonated form, is dialysed versus 10% (v/v) acetic acid in water, then lyophilized. The lyophilized protein is dissolved in a sufficient amount of deaerated 0.1 M HCl to give a protein concentration of 1 mg/mL. The protein/HCl solution contains 5 moles tryptophan/mole fusion protein. CNBr (500 equivalents per equivalent of methionine) is added, and the reaction allowed to proceed for 18 hours, at room temperature in the dark. Large digestion fragments, including the desired modified PE$_{40}$, are then separated from the reaction mixture by gel filtration (e.g., Sephadex G-25) in 25% acetic acid (v/v). Fractions containing the modified PE$_{40}$ are pooled and lyophilized.

In the case of the modified proteins containing cysteine (i.e PE$_{40}$ AB, PE$_{40}$ aB, and PE$_{40}$ Ab) it is necessary to form the requisite disulfide bonds before proceeding with purification. The lyophilized protein is therefore dissolved in a sufficient amount of 50 mM glycine, pH 10.5 to give a UV A$_{280}$ = 0.1. Beta-mercaptoethanol is added to give a 4:1 molar ratio over the theoretical number of S-sulfonate groups present in the protein sample. The reaction is allowed to proceed for 16 hours at 4° C, after which time the solution is dialysed against a 10,000 fold excess of a buffer containing 20 mM Tris, 1 mM EDTA, 100 mM NaCl, pH 8.0.

Fractions from the anion exchange column containing the desired PE$_{40}$ are pooled based on ADP-ribosylation activity and protein content as determined by SDS-PAGE. The pooled fractions are concentrated using a 30,000 molecular weight cutoff membrane (YM-30, Amicon).

The pooled fractions are applied to a 2.6 x 100 cm Sephacryl S-200 gel filtration column (Pharmacia LKB Biotechnology, Inc.), equilibrated in, and eluted with 20 mM Tris, 50 mM NaCl, 1 mM EDTA, pH 8.0 at a flow rate of 0.75 mL/minute. Fractions from the gel filtration chromatography are pooled based on ADP-ribosylation and SDS-PAGE.

Though this procedure yields material sufficiently pure for most purposes, another chromatographic step is included in order to produce highly homogeneous material. This final chromatographic step is high resolution gel filtration, using a 0.75 x 60 cm Bio-Sil TSK-250 column (Bio-Rad). In preparation for chromatography on the TSK-250 column, samples are concentrated on Centriprep-30 devices (Amicon) and protein concentration adjusted to 5 mg/mL. The sample is dissolved in 6 M urea, 100 mM sodium phosphate, 100 mM NaCl, pH 7.1. The column is eluted with 6 M urea, 100 mM sodium phosphate, 100 mM NaCl, pH 7.1, at a flow rate of 0.5 mL/minute. Fractions from the high resolution gel filtration step are pooled based on ADP-ribosylation and SDS-PAGE.

Example 5

Conjugation of EGF to modified PE$_{40}$s and isolation of conjugates

In order to conjugate EGF to modified PE$_{40}$, it is necessary to derivatize both the EGF and PE40 with heterobifunctional agents, so that a covalent connection between the two molecules can be achieved. In preparation for the derivatization, samples of modified PE$_{40}$ are dialyzed against 0.1 M NaCl, 0.1 M sodium phosphate, pH 7.0. Following dialysis, the solution of modified PE$_{40}$ is adjusted to 4 mg/mL PE$_{40}$ using the dialysis buffer, giving a concentration of 100 uM. A sufficient amount of a 20 mM solution of N-succinimidyl 3-(3-pyridyldithio)propionate (SPDP, Pierce) in ethanol is added to the protein solution to give a final concentration of 300 uM SPDP. This concentration represents a 3:1 ratio of SPDP to PE$_{40}$. The derivatization reaction is allowed to proceed at room temperature for 30 minutes, with occasional agitation of the mixture. The reaction is terminated by adding a large excess of glycine (approximately a 50-fold molar excess over the initial amount of SPDP). The resulting 3-(2-pyridyldithio)propionyl-derivative is called PDP-PE$_{40}$. The non-protein reagents are removed from the product by extensive dialysis versus 6 M urea, 0.1 M NaCl, 0.1 M sodium phosphate, pH 7.5. The number of PDP-groups introduced into the modified PE$_{40}$ is determined as described by Carlsson et al., Biochem. J., 173:723-737 1978.

The PDP-EGF derivative is prepared by dissolving lyophilized EGF (Receptor grade, Collaborative Research) in a sufficient amount of 0.1 M NaCl, 0.1 M sodium phosphate, pH 7.0 to give a final concentration of 150 uM EGF. A sufficient amount of a 20 mM solution of SPDP in ethanol is added to the EGF solution to give a final concentration of 450 uM SPDP, representing a 3:1 ratio of SPDP to EGF. The derivatization reaction is allowed to proceed at room temperature for 30 minutes, with occasional agitation of the mixture. The reaction is terminated by adding a large excess of glycine (approximately a 50-fold molar excess over the initial amount of SPDP). The non-protein reagents are removed from the product by extensive dialysis versus 6 M urea, 0.1 M NaCl, 0.1 M sodium phosphate, pH 7.5. The number of PDP-groups introduced into EGF is determined as described by Carlsson et al., Biochem. J., $\underline{173}$:723-737 1978.

Using the derivatives described above, either PDP-PE$_{40}$ or PDP-EGF can be reduced at acidic pH, in order to generate the 3-thiopropionyl derivative, in the presence of the intact, native disulfides (Carlsson et al., supra). However, the preferred strategy is the generation of a free thiol on the modified PE$_{40}$.

PDP-PE$_{40}$ (0.4 ml of a 100 uM solution of PDP-PE$_{40}$ in 6 M urea, 0.1 M NaCl, 0.1 M sodium phosphate, pH 7.5) is dialyzed against several 500 mL changes of a buffer containing 6 M urea, 25 mM sodium acetate, pH 5.5, at 4°C. Following the dialysis, 20 uL of 100 mM dithiothreitol (final concentration 5 mM) is added to the PDP-PE$_{40}$. The reduction is allowed to proceed for 10 minutes at room temperature, and is then terminated by dialysis of the reaction mixture against 6 M urea, 25 mM sodium acetate, 1 mM EDTA, pH 5.5, at 4°C. Dialysis against this buffer is repeated, and then the sample is dialyzed against 0.1 M NaCl, 0.1 M sodium phosphate, pH 7.5. The material generated by these manipulations is called thiopropionyl-PE$_{40}$.

In preparation for conjugation, PDP-EGF (0.8 mL of a 150 uM solution in 6 M urea, 0.1 M NaCl, 0.1 M sodium phosphate, pH 7.5) is dialyzed against several changes of 0.1 M NaCl, 0.1 M sodium phosphate, pH 7.5, at 4°C, to free the sample of urea. Following this dialysis, the PDP-EGF solution and the thiopropionyl-PE$_{40}$ solution are combined and the reaction mixture is incubated at room temperature for 1 hour. The progress of the reaction can be monitored by measuring the release of pyridine-2-thione as described (Carlsson et al., supra). The reaction is terminated by dialysis against several changes of 6 M urea, 0.1 M NaCl, 0.1 M sodium phosphate, pH 7.5, at 4°C.

The conjugates are purified by size exclusion chromatography, using a high resolution 0.75 x 60 cm Bio-Sil TSK-250 column (Bio-Rad). The column is eluted with 6 M urea, 0.1 M sodium phosphate, 0.1 M NaCl, pH 7.1, at a flow rate of 0.5 mL/minute. Fractions from the high resolution gel filtration step are pooled based on ADP-ribosylation and SDS-PAGE.

Example 6

Conjugation of TGF-alpha to modified PE$_{40}$s and isolation of conjugates

Conjugation of TGF-alpha to modified PE$_{40}$s and isolation of conjugates. Conjugates of TGF-alpha and the modified PE$_{40}$ are prepared in a fashion analogous to the EGF-conjugates describe in Example 5.

Example 7

Biologic activites of TGF-alpha - modified PE$_{40}$ conjugates

The relevant biologic activities of the TGF-alpha - modified PE$_{40}$ conjugates are similar to the respective biologic activities of the hybrid TGF-alpha - PE$_{40}$ described in U.S. patent application S.N. 312,540 filed 17 February 1989 by Allen Oliff, Deborah D. Jones and Gwynneth Edwards.

## TABLE 1

pTAC TGF57-PE40

## TABLE 2

ATGGCTGCAGCAGTGGTGTCCCATTTTAATGACTGCCCAGATTCCCACACTCAGTTCTGCTTCCATGGAACATGCAGG

TTTTTGGTGCAGGAGGACAAGCCGGCATGTGTCTGCCATTCTGGGTACGTTGGTGCGCGCTGTGAGCATGCGGACCTC

CTGGCTGCTATGGCCGAAGAGGGCGGCAGCCTGGCCGCGCTGACCGCGCACCAGGCTTGCCACCTGCCGCTGGAGACT

TTCACCCGTCATCGCCAGCCGCGCGGCTGGGAACAACTGGAGCAGTGCGGCTATCCGGTGCAGCGGCTGGTCGCCCTC

TACCTGGCGGCGCGGCTGTCGTGGAACCAGGTCGACCAGGTGATCCGCAACGCCCTGGCCAGCCCCGGCAGCGGCGGC

GACCTGGGCGAAGCGATCCGCGAGCAGCCGGAGCAGGCCCTGGCCCTGACCCTGGCCGCCGCCGAGAGCGAGCGCTTC

GTCCGGCAGGGCACCGGCAACGACGAGGCCGGCGCGGCCAACGCCGACGTGGTGAGCCTGACCTGCCCGGTCGCCGCC

GGTGAATGCGCGGGCCCGGCGGACAGCGGCGACGCCCTGCTGGAGCGCAACTATCCCACTGGCGCGGAGTTCCTCGGC

GACGGCGGCGACGTCAGCTTCAGCACCCGCGGCACGCAGAACTGGACGGTGGAGCGGCTGCTCCAGGCGCACCGCCAA

CTGGAGGAGCGCGGCTATGTGTTCGTCGGCTACCACGGCACCTTCCTCGAAGCGGCGCAAAGCATCGTCTTCGGCGGG

GTGCGCGCGCGCAGCCAGGACCTCGACGCGATCTGGCGCGGTTTCTATATCGCCGGCGATCCGGCGCTGGCCTACGGC

TACGCCCAGGACCAGGAACCCGACGCACGCGGCCGGATCCGCAACGGTGCCCTGCTGCGGGTCTATGTGCCGCGCTCG

AGCCTGCCGGGCTTCTACCGCACCAGCCTGACCCTGGCCGCGCCGGAGGCGGCGGGCGAGGTCGAACGGCTGATCGGC

CATCCGCTGCCGCTGCGCCTGGACGCCATCACCGGCCCCGAGGAGGAAGGCGGGCGCCTGGAGACCATTCTCGGCTGG

CCGCTGGCCGAGCGCACCGTGGTGATTCCCTCGGCGATCCCCACCGACCCGCGCAACGTCGGCGGCGACCTCGACCCG

TCCAGCATCCCCGACAAGGAACAGGCGATCAGCGCCCTGCCGGACTACGCCAGCCAGCCCGGCAAACCGCCGCGCGAG

GACCTGAAGTAA

## TABLE 3

### TGF-alpha-PE$_{40}$ AMINO ACID SEQUENCE

| -4 | -3 | -2 | -1 | TGFα 1 | | | | | | 6 | | | | | | | | | | 16 |
|----|----|----|----|----|----|----|----|----|----|----|----|----|----|----|----|----|----|----|----|----|
| Met | Ala | Ala | Ala | Val | Val | Ser | His | Phe | Asn | Asp | Cys | Pro | Asp | Ser | His | Thr | Gln | Phe | Cys | |

|  |  |  |  |  |  |  |  |  | 26 |  |  |  |  |  |  |  |  |  | 36 |
|----|----|----|----|----|----|----|----|----|----|----|----|----|----|----|----|----|----|----|----|
| Phe | His | Gly | Thr | Cys | Arg | Phe | Leu | Val | Gln | Glu | Asp | Lys | Pro | Ala | Cys | Val | Cys | His | Ser |

|  |  |  |  |  |  |  |  | 46 | TGFα 50 | | | | | PE 252 | | | | | |
|----|----|----|----|----|----|----|----|----|----|----|----|----|----|----|----|----|----|----|----|
| Gly | Tyr | Val | Gly | Ala | Arg | Cys | Glu | His | Ala | Asp | Leu | Leu | Ala | Ala | Met | Ala | Glu | Glu | Gly |

|  |  |  |  |  |  |  |  |  | 263 |  |  |  |  |  |  |  |  |  | 273 |
|----|----|----|----|----|----|----|----|----|----|----|----|----|----|----|----|----|----|----|----|
| Gly | Ser | Leu | Ala | Ala | Leu | Thr | Ala | His | Gln | Ala | Cys | His | Leu | Pro | Leu | Glu | Thr | Phe | Thr |

|  |  |  |  |  |  |  |  |  | 283 |  |  |  |  |  |  |  |  |  | 293 |
|----|----|----|----|----|----|----|----|----|----|----|----|----|----|----|----|----|----|----|----|
| Arg | His | Arg | Gln | Pro | Arg | Gly | Trp | Glu | Gln | Leu | Glu | Gln | Cys | Gly | Tyr | Pro | Val | Gln | Arg |

|  |  |  |  |  |  |  |  |  | 303 |  |  |  |  |  |  |  |  |  | 313 |
|----|----|----|----|----|----|----|----|----|----|----|----|----|----|----|----|----|----|----|----|
| Leu | Val | Ala | Leu | Tyr | Leu | Ala | Ala | Arg | Leu | Ser | Trp | Asn | Gln | Val | Asp | Gln | Val | Ile | Arg |

|  |  |  |  |  |  |  |  |  | 323 |  |  |  |  |  |  |  |  |  | 333 |
|----|----|----|----|----|----|----|----|----|----|----|----|----|----|----|----|----|----|----|----|
| Asn | Ala | Leu | Ala | Ser | Pro | Gly | Ser | Gly | Gly | Asp | Leu | Gly | Glu | Ala | Ile | Arg | Glu | Gln | Pro |

|  |  |  |  |  |  |  |  |  | 343 |  |  |  |  |  |  |  |  |  | 353 |
|----|----|----|----|----|----|----|----|----|----|----|----|----|----|----|----|----|----|----|----|
| Glu | Gln | Ala | Arg | Leu | Ala | Leu | Thr | Leu | Ala | Ala | Ala | Glu | Ser | Glu | Arg | Phe | Val | Arg | Gln |

|  |  |  |  |  |  |  |  |  | 363 |  |  |  |  |  |  |  |  |  | 373 |
|----|----|----|----|----|----|----|----|----|----|----|----|----|----|----|----|----|----|----|----|
| Gly | Thr | Gly | Asn | Asp | Glu | Ala | Gly | Ala | Ala | Asn | Ala | Asp | Val | Val | Ser | Leu | Thr | Cys | Pro |

|  |  |  |  |  |  |  |  |  | 383 |  |  |  |  |  |  |  |  |  | 393 |
|----|----|----|----|----|----|----|----|----|----|----|----|----|----|----|----|----|----|----|----|
| Val | Ala | Ala | Gly | Glu | Cys | Ala | Gly | Pro | Ala | Asp | Ser | Gly | Asp | Ala | Leu | Leu | Glu | Arg | Asn |

|  |  |  |  |  |  |  |  |  | 403 |  |  |  |  |  |  |  |  |  | 413 |
|----|----|----|----|----|----|----|----|----|----|----|----|----|----|----|----|----|----|----|----|
| Tyr | Pro | Thr | Gly | Ala | Glu | Phe | Leu | Gly | Asp | Gly | Gly | Asp | Val | Ser | Phe | Ser | Thr | Arg | Gly |

|  |  |  |  |  |  |  |  |  | 423 |  |  |  |  |  |  |  |  |  | 433 |
|----|----|----|----|----|----|----|----|----|----|----|----|----|----|----|----|----|----|----|----|
| Thr | Gln | Asn | Trp | Thr | Val | Glu | Arg | Leu | Leu | Gln | Ala | His | Arg | Gln | Leu | Glu | Glu | Arg | Gly |

|  |  |  |  |  |  |  |  |  | 443 |  |  |  |  |  |  |  |  |  | 453 |
|----|----|----|----|----|----|----|----|----|----|----|----|----|----|----|----|----|----|----|----|
| Tyr | Val | Phe | Val | Gly | Tyr | His | Gly | Thr | Phe | Leu | Glu | Ala | Ala | Gln | Ser | Ile | Val | Phe | Gly |

|  |  |  |  |  |  |  |  |  | 463 |  |  |  |  |  |  |  |  |  | 473 |
|----|----|----|----|----|----|----|----|----|----|----|----|----|----|----|----|----|----|----|----|
| Gly | Val | Arg | Ala | Arg | Ser | Gln | Asp | Leu | Asp | Ala | Ile | Trp | Arg | Gly | Phe | Tyr | Ile | Ala | Gly |

## TABLE 3 CONT'D

TGF-alpha-PE$_{40}$ AMINO ACID SEQUENCE

| | 483 | | | | | | | | | | | | | | | | 493 | | |
|---|---|---|---|---|---|---|---|---|---|---|---|---|---|---|---|---|---|---|---|
| Asp | Pro | Ala | Leu | Ala | Tyr | Gly | Tyr | Ala | Gln | Asp | Gln | Glu | Pro | Asp | Ala | Arg | Gly | Arg | Ile |

503       513

Arg Asn Gly Ala Leu Leu Arg Val Tyr Val Pro Arg Ser Ser Leu Pro Gly Phe Tyr Arg

523       533

Thr Ser Leu Thr Leu Ala Ala Pro Glu Ala Ala Gly Glu Val Glu Arg Leu Ile Gly His

543       553

Pro Leu Pro Leu Arg Leu Asp Ala Ile Thr Gly Pro Glu Glu Glu Gly Gly Arg Leu Glu

563       573

Thr Ile Leu Gly Trp Pro Leu Ala Glu Arg Thr Val Val Ile Pro Ser Ala Ile Pro Thr

583       593

Asp Pro Arg Asn Val Gly Gly Asp Leu Asp Pro Ser Ser Ile Pro Asp Lys Glu Gln Ala

603       613

Ile Ser Ala Leu Pro Asp Tyr Ala Ser Gln Pro Gly Lys Pro Pro Arg Glu Asp Leu Lys

## TABLE 4

### TGF-alpha-PE$_{40}$-aB AMINO ACID SEQUENCE

| -4 | -3 | -2 | -1 | TGFa$^1$ | | | | | 6 | | | | | | | | | | 16 |
|---|---|---|---|---|---|---|---|---|---|---|---|---|---|---|---|---|---|---|---|
| Met | Ala | Ala | Ala | Val | Val | Ser | His | Phe | Asn | Asp | Cys | Pro | Asp | Ser | His | Thr | Gln | Phe | Cys |

| | | | | | | | | | 26 | | | | | | | | | | 36 |
|---|---|---|---|---|---|---|---|---|---|---|---|---|---|---|---|---|---|---|---|
| Phe | His | Gly | Thr | Cys | Arg | Phe | Leu | Val | Gln | Glu | Asp | Lys | Pro | Ala | Cys | Val | Cys | His | Ser |

| | | | | | | | | | 46 | | TGFa$^{50}$ | | | | | | PE$^{252}$ | | |
|---|---|---|---|---|---|---|---|---|---|---|---|---|---|---|---|---|---|---|---|
| Gly | Tyr | Val | Gly | Ala | Arg | Cys | Glu | His | Ala | Asp | Leu | Leu | Ala | Ala | Met | Ala | Glu | Glu | Gly |

| | | | | | | | | | 263 | | | | | | | | | | 273 |
|---|---|---|---|---|---|---|---|---|---|---|---|---|---|---|---|---|---|---|---|
| Gly | Ser | Leu | Ala | Ala | Leu | Thr | Ala | His | Gln | Ala | Ala | His | Leu | Pro | Leu | Glu | Thr | Leu | Thr |

| | | | | | | | | | 283 | | | | | | | | | | 293 |
|---|---|---|---|---|---|---|---|---|---|---|---|---|---|---|---|---|---|---|---|
| Arg | His | Arg | Gln | Pro | Arg | Gly | Trp | Glu | Gln | Leu | Glu | Gln | Ala | Gly | Tyr | Pro | Val | Gln | Arg |

| | | | | | | | | | 303 | | | | | | | | | | 313 |
|---|---|---|---|---|---|---|---|---|---|---|---|---|---|---|---|---|---|---|---|
| Leu | Val | Ala | Leu | Tyr | Leu | Ala | Ala | Arg | Leu | Ser | Trp | Asn | Gln | Val | Asp | Gln | Val | Ile | Arg |

| | | | | | | | | | 323 | | | | | | | | | | 333 |
|---|---|---|---|---|---|---|---|---|---|---|---|---|---|---|---|---|---|---|---|
| Asn | Ala | Leu | Ala | Ser | Pro | Gly | Ser | Gly | Gly | Asp | Leu | Gly | Glu | Ala | Ile | Arg | Glu | Gln | Pro |

| | | | | | | | | | 343 | | | | | | | | | | 353 |
|---|---|---|---|---|---|---|---|---|---|---|---|---|---|---|---|---|---|---|---|
| Glu | Gln | Ala | Arg | Leu | Ala | Leu | Thr | Leu | Ala | Ala | Ala | Glu | Ser | Glu | Arg | Phe | Val | Arg | Gln |

| | | | | | | | | | 363 | | | | | | | | | | 373 |
|---|---|---|---|---|---|---|---|---|---|---|---|---|---|---|---|---|---|---|---|
| Gly | Thr | Gly | Asn | Asp | Glu | Ala | Gly | Ala | Ala | Asn | Ala | Asp | Val | Val | Ser | Leu | Thr | Cys | Pro |

| | | | | | | | | | 383 | | | | | | | | | | 393 |
|---|---|---|---|---|---|---|---|---|---|---|---|---|---|---|---|---|---|---|---|
| Val | Ala | Ala | Gly | Glu | Cys | Ala | Gly | Pro | Ala | Asp | Ser | Gly | Asp | Ala | Leu | Leu | Glu | Arg | Asn |

| | | | | | | | | | 403 | | | | | | | | | | 413 |
|---|---|---|---|---|---|---|---|---|---|---|---|---|---|---|---|---|---|---|---|
| Tyr | Pro | Thr | Glu | Ala | Glu | Phe | Leu | Gly | Asp | Gly | Gly | Asp | Val | Ser | Phe | Ser | Thr | Arg | Gly |

| | | | | | | | | | 423 | | | | | | | | | | 433 |
|---|---|---|---|---|---|---|---|---|---|---|---|---|---|---|---|---|---|---|---|
| Thr | Gln | Asn | Trp | Thr | Val | Glu | Arg | Leu | Leu | Gln | Ala | His | Arg | Gln | Leu | Glu | Glu | Arg | Gly |

| | | | | | | | | | 443 | | | | | | | | | | 453 |
|---|---|---|---|---|---|---|---|---|---|---|---|---|---|---|---|---|---|---|---|
| Tyr | Val | Phe | Val | Gly | Tyr | His | Gly | Thr | Phe | Leu | Glu | Ala | Ala | Gln | Ser | Ile | Val | Phe | Gly |

| | | | | | | | | | 463 | | | | | | | | | | 473 |
|---|---|---|---|---|---|---|---|---|---|---|---|---|---|---|---|---|---|---|---|
| Gly | Val | Arg | Ala | Arg | Ser | Gln | Asp | Leu | Asp | Ala | Ile | Trp | Arg | Gly | Phe | Tyr | Ile | Ala | Gly |

## TABLE 4 CONT'D

### TGF-alphs-PE$_{40}$ aB AMINO ACID SEQUENCE

```
                         483                              493
Asp Pro Ala Leu Ala Tyr Gly Tyr Ala Gln Asp Gln Glu Pro Asp Ala Arg Gly Arg Ile
                         503                              513
Arg Asn Gly Ala Leu Leu Arg Val Tyr Val Pro Arg Ser Ser Leu Pro Gly Phe Tyr Arg
                         523                              533
Thr Ser Leu Thr Leu Ala Ala Pro Glu Ala Ala Gly Glu Val Glu Arg Leu Ile Gly His
                         543                              553
Pro Leu Pro Leu Arg Leu Asp Ala Ile Thr Gly Pro Glu Glu Glu Gly Gly Arg Leu Glu
                         563                              573
Thr Ile Leu Gly Trp Pro Leu Ala Glu Arg Thr Val Val Ile Pro Ser Ala Ile Pro Thr
                         583                              593
Asp Pro Arg Asn Val Gly Gly Asp Leu Asp Pro Ser Ser Ile Pro Asp Lys Glu Gln Ala
                         603                              613
Ile Ser Ala Leu Pro Asp Tyr Ala Ser Gln Pro Gly Lys Pro Pro Arg Glu Asp Leu Lys
```

## TABLE 5

TGF-alpha-PE$_{40}$ Ab AMINO ACID SEQUENCE

```
 -4  -3  -2  -1  |TGFα 1                       6                                16
 Met Ala Ala Ala Val Val Ser His Phe Asn Asp Cys Pro Asp Ser His Thr Gln Phe Cys

                                     26                                      36
 Phe His Gly Thr Cys Arg Phe Leu Val Gln Glu Asp Lys Pro Ala Cys Val Cys His Ser
                                     46          TGFα 50 |              PE 252
 Gly Tyr Val Gly Ala Arg Cys Glu His Ala Asp Leu Leu Ala Ala Met Ala Glu Glu Gly
                                    263                                     273
 Gly Ser Leu Ala Ala Leu Thr Ala His Gln Ala Cys His Leu Pro Leu Glu Thr Phe Thr
                                    283                                     293
 Arg His Arg Gln Pro Arg Gly Trp Glu Gln Leu Glu Gln Cys Gly Tyr Pro Val Gln Arg
                                    303                                     313
 Leu Val Ala Leu Tyr Leu Ala Ala Arg Leu Ser Trp Asn Gln Val Asp Gln Val Ile Arg
                                    323                                     333
 Asn Ala Leu Ala Ser Pro Gly Ser Gly Gly Asp Leu Gly Glu Ala Ile Arg Glu Gln Pro
                                    343                                     353
 Glu Gln Ala Arg Leu Ala Leu Thr Leu Ala Ala Ala Glu Ser Glu Arg Phe Val Arg Gln
                                    363                                     373
 Gly Thr Gly Asn Asp Glu Ala Gly Ala Ala Asn Ala Asp Val Val Thr Leu Thr Ala Pro
                                    383                                     393
 Val Ala Ala Gly Glu Ala Ala Gly Pro Ala Asp Ser Gly Asp Ala Leu Leu Glu Arg Asn
                                    403                                     413
 Tyr Pro Thr Gly Ala Glu Phe Leu Gly Asp Gly Gly Asp Val Ser Phe Ser Thr Arg Gly
                                    423                                     433
 Thr Gln Asn Trp Thr Val Glu Arg Leu Leu Gln Ala His Arg Gln Leu Glu Glu Arg Gly
                                    443                                     453
 Tyr Val Phe Val Gly Tyr His Gly Thr Phe Leu Glu Ala Ala Gln Ser Ile Val Phe Gly
                                    463                                     473
 Gly Val Arg Ala Arg Ser Gln Asp Leu Asp Ala Ile Trp Arg Gly Phe Tyr Ile Ala Gly
```

## TABLE 5 CONT'D

### TGF-alpha-PE$_{40}$ Ab AMINO ACID SEQUENCE

```
                                 483                                      493
        Asp Pro Ala Leu Ala Tyr Gly Tyr Ala Gln Asp Gln Glu Pro Asp Ala Arg Gly Arg Ile
                                 503                                      513
        Arg Asn Gly Ala Leu Leu Arg Val Tyr Val Pro Arg Ser Ser Leu Pro Gly Phe Tyr Arg
                                 523                                      533
        Thr Ser Leu Thr Leu Ala Ala Pro Glu Ala Ala Gly Glu Val Glu Arg Leu Ile Gly His
                                 543                                      553
        Pro Leu Pro Leu Arg Leu Asp Ala Ile Thr Gly Pro Glu Glu Glu Gly Gly Arg Leu Glu
                                 563                                      573
        Thr Ile Leu Gly Trp Pro Leu Ala Glu Arg Thr Val Val Ile Pro Ser Ala Ile Pro Thr
                                 583                                      593
        Asp Pro Arg Asn Val Gly Gly Asp Leu Asp Pro Ser Ser Ile Pro Asp Lys Glu Gln Ala
                                 603                                      613
        Ile Ser Ala Leu Pro Asp Tyr Ala Ser Gln Pro Gly Lys Pro Pro Arg Glu Asp Leu Lys
```

## TABLE 6

### TGF-alpha-PE$_{40}$ ab AMINO ACID SEQUENCE

-4  -3  -2  -1  [TGFα$^1$]                6                                    16

Met Ala Ala Ala Val Val Ser His Phe Asn Asp Cys Pro Asp Ser His Thr Gln Phe Cys

26                                    36

Phe His Gly Thr Cys Arg Phe Leu Val Gln Glu Asp Lys Pro Ala Cys Val Cys His Ser

46        TGFα$^{50}$ |                [PE$^{252}$]

Gly Tyr Val Gly Ala Arg Cys Glu His Ala Asp Leu Leu Ala Ala Met Ala Glu Glu Gly

263                                    273

Gly Ser Leu Ala Ala Leu Thr Ala His Gln Ala Ala His Leu Pro Leu Glu Thr Leu Thr

283                                    293

Arg His Arg Gln Pro Arg Gly Trp Glu Gln Leu Glu Gln Ala Gly Tyr Pro Val Gln Arg

303                                    313

Leu Val Ala Leu Tyr Leu Ala Ala Arg Leu Ser Trp Asn Gln Val Asp Gln Val Ile Arg

323                                    333

Asn Ala Leu Ala Ser Pro Gly Ser Gly Gly Asp Leu Gly Glu Ala Ile Arg Glu Gln Pro

343                                    353

Glu Gln Ala Arg Leu Ala Leu Thr Leu Ala Ala Ala Glu Ser Glu Arg Phe Val Arg Gln

363                                    373

Gly Thr Gly Asn Asp Glu Ala Gly Ala Ala Asn Ala Asp Val Val Thr Leu Thr Ala Pro

383                                    393

Val Ala Ala Gly Glu Ala Ala Gly Pro Ala Asp Ser Gly Asp Ala Leu Leu Glu Arg Asn

403                                    413

Tyr Pro Thr Gly Ala Glu Phe Leu Gly Asp Gly Gly Asp Val Ser Phe Ser Thr Arg Gly

423                                    433

Thr Gln Asn Trp Thr Val Glu Arg Leu Leu Gln Ala His Arg Gln Leu Glu Glu Arg Gly

443                                    453

Tyr Val Phe Val Gly Tyr His Gly Thr Phe Leu Glu Ala Ala Gln Ser Ile Val Phe Gly

463                                    473

Gly Val Arg Ala Arg Ser Gln Asp Leu Asp Ala Ile Trp Arg Gly Phe Tyr Ile Ala Gly

TABLE 6 CONT'D

TGF-alpha-PE$_{40}$ ab AMINO ACID SEQUENCE

```
                                   483                                      493
     Asp Pro Ala Leu Ala Tyr Gly Tyr Ala Gln Asp Gln Glu Pro Asp Ala Arg Gly Arg Ile
                                   503                                      513
     Arg Asn Gly Ala Leu Leu Arg Val Tyr Val Pro Arg Ser Ser Leu Pro Gly Phe Tyr Arg
                                   523                                      533
     Thr Ser Leu Thr Leu Ala Ala Pro Glu Ala Ala Gly Glu Val Glu Arg Leu Ile Gly His
                                   543                                      553
     Pro Leu Pro Leu Arg Leu Asp Ala Ile Thr Gly Pro Glu Glu Glu Gly Gly Arg Leu Glu
                                   563                                      573
     Thr Ile Leu Gly Trp Pro Leu Ala Glu Arg Thr Val Val Ile Pro Ser Ala Ile Pro Thr
                                   583                                      593
     Asp Pro Arg Asn Val Gly Gly Asp Leu Asp Pro Ser Ser Ile Pro Asp Lys Glu Gln Ala
                                   603                                      613
     Ile Ser Ala Leu Pro Asp Tyr Ala Ser Gln Pro Gly Lys Pro Pro Arg Glu Asp Leu Lys
```

## Claims

1. A polypeptide selected from the group consisting of PE$_{40}$ aB, PE$_{40}$ Ab, or PE$_{40}$ ab wherein 'A' indicates the presence of cysteine at positions 265 and 287 of the amino acid sequence of PE$_{40}$; 'a' indicates the presence of an amino acid other than cysteine or the absence of an amino acid at positions 265 and 287; 'B' indicates the presence of cysteine at positions 372 and 379; and 'b' indicates the presence of an amino acid other than cysteine or the absence of an amino acid at positions 372 and 379.

2. A polypeptide according to claim 1 that is PE$_{40}$ aB.

3. A polypeptide according to claim 1 that is PE$_{40}$ Ab.

4. A polypeptide according to claim 1 that is PE$_{40}$ ab.

5. A polypeptide according to claim 1 wherein a is missing.

6. A polypeptide according to claim 1 wherein b is missing.

7. A polypeptide according to claim 1 wherein both a and b are missing.

8. A polypeptide according to claim 1 wherein a is an amino acid other than Cys.

9. A polypeptide according to claim 8 wherein a is Ala.

10. A polypeptide according to claim 1 wherein b is an amino acid other than Cys.

11. A polypeptide according to claim 10 wherein b is Ala.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | US-A-4 545 985 (PASTAN) --- | | C 12 N 15/31<br>C 12 N 15/62<br>A 61 K 47/48 |
| D,A | EP-A-0 261 671 (PASTAN) --- | | |
| A | WO-A-8 702 987 (MURPHY) --- | | |
| D,A | PROCEEDINGS OF THE NATIONAL ACADEMY OF SCIENCES USA, vol. 81, May 1984, pages 2645-2649; G.L. GRAY et al.: "Cloning, nucleotide sequence, and expression in Escherichia coli of the exotoxin a structural gene of Pseudomonas aeruginosa" --- | | |
| P,X | THE JOURNAL OF BIOLOGICAL CHEMISTRY, vol. 264, no. 24, 25th August 1989, pages 14256-14261, US; C.B. SIEGALL et al.: "Functional analysis of domains II,Ib, and III of Pseudomonas exotoxin" * Page 14260, column 2, lines 3-6 * --- | 1,2,8 | |
| P,X | INFECTION AND IMMUNITY, vol. 57, no. 7, July 1989, pages 1873-1878, American Society for Microbiology; I.H. MADSHUS et al.: "Effects of eliminating a disulfide bridge within domain II of Pseudomonas aeruginosa exotoxin A" * Whole document * --- | 1,2,8 | TECHNICAL FIELDS SEARCHED (Int. Cl.5)<br><br>C 12 N<br>A 61 K |
| P,X | MOLECULAR AND CELLULAR BIOLOGY, vol. 9, no. 7, July 1989, pages 2860-2867; G.M. EDWARDS et al.: "Epidermal growth actor receptor binding 1s affected by structural determinants in the toxin domain of transforming growth factor-alpha-Pseudomonas exotoxin fusion proteins" ----- | 1-11 | |

The present search report has been drawn up for all claims

| Place of search | Date of completion of the search | Examiner |
|---|---|---|
| THE HAGUE | 25-06-1990 | SKELLY J.M. |